# NOUVEAU FASCICULE DE BREVET EUROPEEN

(11) **EP 1 328 269 B2**
(45) Date de publication et mention de la décision concernant l'opposition: **30.07.2008**
(45) Mention de la délivrance du brevet: 26.05.2004
(21) Numéro de dépôt: 01974413.5
(22) Date de dépôt: 01.10.2001
(51) Int. Cl.: A61K 31/397, A61K 31/137, A61P 3/04

(54) **ASSOCIATION D'UN ANTAGONISTE DU RECEPTEUR CB1 ET DE LA SIBUTRAMINE POUR LE TRAITEMENT DE L'OBESITE**
ZUSAMMENSETZUNGEN ZUR BEHANDLUNG VON FETTLEIBIGKEIT, WELCHE ANTAGONISTEN DER CB1-REZEPTOREN UND SIBUTRAMINE ENTHALTEN
ASSOCIATION OF A CB1 RECEPTOR ANTAGONIST AND SIBUTRAMIN FOR TREATING OBESITY

(30) Priorité: 04.10.2000 FR 0012646
(43) Date de publication de la demande: 23.07.2003
(73) Titulaire: Aventis Pharma S.A., 92160 Antony (FR)
(72) Inventeur: PIOT-GROSJEAN, Odile, F-94600 CHOISY LE ROIS (FR); PICAUT, Philippe, F-92260 FONTENAY AUX ROSES (FR); PETITET, François, F-94000 CRETEIL (FR)
(74) Mandataire: Caen, Thierry Alain
(86) Numéro de dépôt international: PCT/FR2001/003022
(87) Numéro de publication internationale: WO 2002/028346

(56) Documents cités:
- EP-A- 0 656 354
- EP-A- 0 920 864
- WO-A-00/15609
- WO-A-01/64633
- WO-A-01/64634
- WO-A-90/06110
- WO-A-96/02248
- WO-A-98/18481
- WO-A-98/32441
- US-A1- 2002 035 102
- US-B1- 6 344 474
- PROIETTO, JOSEPH ET AL: "Novel anti-obesity drugs" EXPERT OPIN. INVEST. DRUGS (2000), 9(6), 1317-1326, 2000, XP001004696
- J. Hirsch, BMJ, 1998, 317:1136-1138
- Nakazi et al., Naumyn-Schmiedeberg's Arch. Pharmacol., 2000; 361:19-24
- Déclaration by Bret J. Musser
- Petit Robert 1, dictionnaire alphabétique et analogique de la langue francaise, p. 85
- J.J. Peterson; GlaxoSmithkline - Technical Report
- PNAS, vol. 100, n° 13
- Food and Drug Administration, Subchapter D

## Description

La présente invention concerne l'association d'un antagoniste du récepteur CB1 et de sibutramine, les compositions pharmaceutiques les contenant et leur utilisation pour le traitement de l'obésité.

Les antagonistes du récepteur CB1 sont connus pour leur action sur la prise alimentaire et leur utilisation comme anorexigène (G. COLOMBO et coll., Life Sciences, 63 (8), 113-117 (1998); J. SIAMAND et coll., Behavioural Pharmacol., 9, 179-181 (1998)).

La sibutramine (BTS 54524; N-{1-[1-(4-chlorophényl)cyclobutyl]-3-méthylbutyl}-N,N-diméthylamine; Mendia^{R}, Réductil^{R}), son hydrate et ses sels pharmaceutiquement acceptables et notamment son chlorhydrate diminue la prise alimentaire et est utile pour le traitement de l'obésité (WO90/061110; D.H. RYAN et coll., Obesity Research, 3 (4), 553 (1995); H.C. JACKSON et coll., British Journal of Pharmacology, 121, 1758 (1997); G. FANGHANEL et Coll., Inter. J. Obes., 24 (2), 144 (2000); G.A. BRAY et coll., Obes. Res., 7 (2) 189 (1999)).

Il a maintenant été trouvé que l'association de sibutramine, son hydrate et ses sels pharmaceutiquement acceptables et d'un antagoniste du récepteur CB1 consistant en le 1-[bis(4-chlorophényl)méthyl]-3-[(3,5-difluorophényl)(méthylsulfonyl)méthylène]azétidine et ses isomères optiques et les sels pharmaceutiquement acceptable présente un effet de synergie dans la réduction de la consommation alimentaire et est donc utile dans le traitement de l'obésité.

L'association peut également contenir plusieurs antagonistes du récepteur CB1.

Cet antagoniste CB1, est décrit dans WO 00/15609.

Comme exemples de sels pharmaceutiquement acceptables, peuvent être cités les sels suivants : benzènesulfonate, bromhydrate, chlorhydrate, citrate, éthanesulfonate, fumarate, gluconate, iodate, iséthionate, maléate, méthanesulfonate, méthylène-bis-β-oxynaphtoate, nitrate, oxalate, pamoate, phosphate, salicylate, succinate, sulfate, tartrate, théophyllinacétate et p-toluènesulfonate.

L'effet de synergie de l'association de sibutramine et de l'antagoniste CB1 de l'invention dans la prise alimentaire a été déterminé selon le protocole suivant :

Des rats Zucker obèses Fa/fa âgés de 7 semaines et provenant de Iffa-Credo, France ont été utilisés dans cette étude. Les rats sont hébergés dans des cages individuelles et pesés tous les jours entre 8 et 10 heures le matin. La quantité de nourriture est également pesée chaque matin à la même heure. Cette nourriture (M20, Pietrement, France) est changée chaque jour et les rats y ont librement accès durant 24 heures. Pendant une semaine tous les rats sont traités par le véhicule (miglyol 812N et méthylcellulose 0,5%/polysorbate 80 0,2%) en deux administrations consécutives. A partir du 8ème jour les rats sont traités par le véhicule, l'antagoniste CB1 (1-[bis(4-chlorophényl)méthyl]-3-[(3,5-difluorophényl)(méthylsulfonyl)méthylène]azétidine) ou la sibutramine par voie orale (voir table ci-dessous).

La sibutramine est mise en solution dans une solution de méthylcellulose 0,5%/polysorbate 80 0,2% et administrée à la dose de 3 mg/kg; l'antagoniste CB1 est mis en solution dans du miglyol 812N (Hüls, Allemagne) à la dose de 0,6 mg/kg, les deux produits sont administrés sous un volume de 1 ml/kg. Chaque groupe est constitué de 12 à 14 animaux. Les groupes suivants sont constitués et les animaux sont traités pendant cinq jours à raison de deux administrations consécutives chaque jour.

| GROUPE | Première **administration** | Deuxième administration |
|---|---|---|
| **1** | miglyol | methylcellulose 0,5 % / |
| "groupe véhicule" | | polysorbate 0,2% |
| **2** | miglyol | sibutramine 3 mg/kg |
| "groupe sibutramine" | | |
| **3** | antagoniste CB1 | methylcellulose 0,5 % / |
| "groupe antagoniste CB1" | 0,6mg/kg | polysorbate 0,2% |
| **4** | antagoniste CB1 | sibutramine 3 mg/kg |
| "groupe association" | 0,6mg/kg | |

Chaque jour, la consommation alimentaire de chaque animal est mesurée. Les résultats sont exprimés en quantité moyenne de nourriture consommée durant les 5 jours de traitement. Les résultats obtenus sont mentionnés dans le tableau ci-dessous.

| **Traitement** | **Consommation alimentaire durant les 5 jours de traitement (g)** |
|---|---|
| Véhicules | 25,52 ± 0,30 |
| sibutramine 3 mg/kg | 24,60 ± 0,32* |
| antagoniste CB1 0,6 mg/kg | 24,00 ± 0,24** |
| sibutramine (3 mg/kg) + antagoniste CB1 (0,6 mg/kg) | 22,76 ± 0,02*** |

| | |
|---|---|
| * p <0,05 | |
| ** p <0,01 | |
| *** p <0,0001 | |

Les résultats démontrent que chez les animaux recevant l'association sibutramine et antagoniste CB1 la diminution de la consommation alimentaire est très supérieure a celle des animaux traités soit par la sibutramine seule soit par l'antagoniste CB1 seul.

Les composés de l'association peuvent être employés par voie orale, parentérale, transdermale ou rectale soit simultanément soit séparément soit de façon étalée dans le temps.

La présente invention concerne également les compositions pharmaceutiques contenant l'association de sibutramine, son hydrate ou un de ses sels pharmaceutiquement acceptables et dudit antagoniste du récepteur CB1 à l'état pur ou avec un ou plusieurs diluants et/ou adjuvants compatibles et pharmacologiquement acceptables et/ou éventuellement en association avec un autre produit pharmaceutiquement compatible et physiologiquement actif pour un usage soit simultané, soit séparé, soit étalé dans le temps.

Comme compositions solides pour administration orale, peuvent être utilisés des comprimés, des pilules, des poudres (capsules de gélatine, cachets) ou des granulés. Dans ces compositions, les principes actifs sont mélangés à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semisynthétiques ou des polyéthylèneglycols.

Les compositions pharmaceutiques contiennent généralement 0,5 à 10 mg de sibutramine et 0,1 à 200 mg de l'antagoniste CB1.

La présente invention concerne également l' association selon l'invention pour l'obtention d'un médicament destiné au traitement de l'obésité. Les doses dépendent de l'effet recherché, de la durée du traitement et de la voie d'administration utilisée; elles sont généralement de 1 à 15 mg par jour par voie orale pour un adulte de sibutramine et de 0,10 à 500 mg par jour par voie orale pour un adulte de l'antagoniste CB1.

D'une façon générale, le médecin déterminera la posologie appropriée en fonction de l'âge, du poids et de tous les autres facteurs propres au sujet à traiter.

## Revendications

1. Association d'un antagoniste CB1 consistant en le
1-[bis(4-chlorophényl)méthyl]-3-[(3,5-difluorophényl)(méthylsulfonyl)méthylène]azétidine,
ses isomères optiques et ses sels pharmaceutiquement acceptables,
et de sibutramine, son hydrate ou un de ses sels pharmaceutiquement acceptables.

2. Composition pharmaceutique contenant un antagoniste CB1 consistant en le
1-[bis(4-chlorophényl)méthyl]-3-[(3,5-difluorophényl)(méthylsulfonyl)méthylène]azétidine,
ses isomères optiques et ses sels pharmaceutiquement acceptables,
et la sibutramine, son hydrate ou un de ses sels pharmaceutiquement acceptables à l'état pur ou avec un ou plusieurs diluants et/ou adjuvants compatibles et pharmacologiquement acceptables et/ou éventuellement en association avec un autre produit pharmaceutiquement compatible et physiologiquement actif.

3. Utilisation de l'association d'un antagoniste CB1 consistant en le
1-[bis(4-chlorophényl)méthyl]-3-[(3,5-difluorophényl)(méthylsulfonyl)méthylène]azétidine,
ses isomères optiques et ses sels pharmaceutiquement acceptables,
et de sibutramine, son hydrate ou un de ses sels pharmaceutiquement acceptables, pour la préparation d'un médicament destiné au traitement de l'obésité.

4. Utilisation de l'association d'un antagoniste CB1 consistant en
1-[bis(4-chlorophényl)méthyl]-3-[(3,5-difluorophényl)(méthylsulfonyl)méthylène]azétidine,
ses isomères optiques et ses sels pharmaceutiquement acceptables,
et de sibutramine, son hydrate ou un de ses sels pharmaceutiquement acceptables, pour la préparation d'un médicament destiné à réduire la consommation alimentaire.

## Claims

1. Combination of a CB1 antagonist consisting of 1-[bis(4-chlorophenyl)methyl]-3-[(3,5-difluorophenyl)-(methylsulphonyl)methylene)azetidine, its optical isomers and its pharmaceutically acceptable salts and of sibutramine, its hydrate or one of its pharmaceutically acceptable salts.

2. Pharmaceutical composition comprising a CB1 antagonist consisting of 1-[bis(4-chlorophenyl)methyl]-3-[(3,5-difluorophenyl)(methylsulphonyl)methylene)-azetidine, its optical isomers and its pharmaceutically acceptable salts and sibutramine, its hydrate or one of its pharmaceutically acceptable salts in a pure state or with one or more compatible and pharmacologically acceptable diluents and/or adjuvants and/or optionally in combination with another pharmaceutically compatible and physiologically active product.

3. Use of the combination of a CB1 antagonist consisting of 1-[bis(4-chlorophenyl)methyl]-3-[(3,5-difluorophenyl)(methylsulphonyl)methylene)azetidine, its optical isomers and its pharmaceutically acceptable salts and of sibutramine, its hydrate or one of its pharmaceutically acceptable salts in the preparation of a medicament intended for the treatment of obesity.

4. Use of the combination of a CB1 antagonist consisting of 1-[bis(4-chlorophenyl)methyl]-3-[(3,5-difluorophenyl)(methylsulphonyl)methylene)azetidine, its optical isomers and its pharmaceutically acceptable salts and of sibutramine, its hydrate or one of its pharmaceutically acceptable salts in the preparation of a medicament intended to reduce food consumption.

## Patentansprüche

1. Kombination eines CB1-Antagonisten bestehend aus 1-[Bis(4-chlorphenyl)methyl]-3-[(3,5-difluorphenyl)(methylsulphonyl)methylen]azetidin, seinen optischen Isomeren und seinen pharmazeutisch annehmbaren Salzen,
und Sibutramin, seinem Hydrat oder einem seiner pharmazeutisch annehmbaren Salze.

2. Pharmazeutische Zusammensetzungen enthaltend einen CB1-Antagonisten bestehend aus 1-[Bis(4-chlorphenyl)methyl]-3-[(3,5-difluorphenyl)(methylsulphonyl)methylen]azetidin, seinen optischen Isomeren und seinen pharmazeutisch annehmbaren Salzen
und Sibutramin, seinem Hydrat oder einem seiner pharmazeutisch annehmbaren Salze im reinen Zustand oder mit einem oder mehreren kompatiblen und pharmazeutisch annehmbaren Verdünnungsmitteln und/oder Hilfsstoffen und/oder gegebenenfalls in Kombination mit einem anderen pharmazeutisch kompatiblen und physiologisch aktiven Produkt.

3. Verwendung der Kombination eines CB1-Antagonisten bestehend aus 1-[Bis(4-chlorphenyl)methyl]-3-[(3,5-difluorphenyl)(methylsulphonyl)methylen]-azetidin, seinen optischen Isomeren und seinen pharmazeutisch annehmbaren Salzen
und Sibutramin, seinem Hydrat oder einem seiner pharmazeutisch annehmbaren Salze für die Herstellung eines Arzneimittels zur Behandlung von Fettleibigkeit.

4. Verwendung der Kombination eines CB1-Antagonisten bestehend aus 1-[Bis(4-chlorphenyl)methyl]-3-[(3,5-difluorphenyl)(methylsulphonyl)methylen]-azetidin, seinen optischen Isomeren und seinen pharmazeutisch annehmbaren Salzen
und Sibutramin, seinem Hydrat oder einem seiner pharmazeutisch annehmbaren Salze für die Herstellung eines Arzneimittels zur Reduktion der Nahrungsaufnahme.
